Europäisches Patentamt

European Patent Office

Office européen des brevets

(11) Veröffentlichungsnummer: **0 518 113 A1**

## (12) EUROPÄISCHE PATENTANMELDUNG

(21) Anmeldenummer: **92108798.7**

(51) Int. Cl.5: **A61K 31/21**, A61K 9/70

(22) Anmeldetag: **25.05.92**

(30) Priorität: **10.06.91 DE 4118891**

(43) Veröffentlichungstag der Anmeldung:
**16.12.92 Patentblatt 92/51**

(84) Benannte Vertragsstaaten:
**PT**

(71) Anmelder: **SCHWARZ PHARMA AG**
**Alfred-Nobel-Strasse 10**
**W-4019 Monheim(DE)**
Anmelder: **LTS Lohmann Therapie-Systeme**
**GmbH & Co. KG**
**Irlicherstrasse 55**
**W-5450 Neuwied 12(DE)**

(72) Erfinder: **Beutner, Dieter, Dr. rat. nat.**
**Lortzingweg 52**
**W-4019 Monheim(DE)**
Erfinder: **Knobelsdorff v., Henning, Dipl.-Ing.**
**Rüsterstrasse 40**
**W-5300 Bonn 3(DE)**
Erfinder: **Wolff, Hans-Michael, Dr. rer. nat.**
**Richard-Wagner-Strasse 2**
**W-4019 Monheim(DE)**
Erfinder: **Hoffmann, Hans-Rainer, Dr. rer. nat.**
**Burghofstrasse 123**
**W-5450 Neuwied 22(DE)**
Erfinder: **Meconi, Reinhold, Dipl.-Ing.**
**Alemannenstrasse 42**
**W-5450 Neuwied 11(DE)**
Erfinder: **Klein, Robert Peter**
**Wikingerstrasse 3**
**W-5450 Neuwied 11(DE)**

(74) Vertreter: **Cohausz & Florack Patentanwälte**
**Postfach 14 01 61 Schumannstrasse 97**
**W-4000 Düsseldorf 1(DE)**

(54) **Nitroglycerin-Pflaster und Verfahren zu seiner Herstellung.**

(57) Die Erfindung betrifft ein Hautpflaster zur transdermalen Verabreichung von Nitroglycerin, bestehend neben einer Trägerfolie und einer abziehbaren Schutzfolie aus einer Nitroglycerin enthaltenden besonderen Klebemasse auf Basis eines vernetzten Acrylat-Vinylacetat-Copolymerisats, dessen zur Polymerisation eingesetztes Monomerengemisch 21 bis 40 Gew.-% Vinylacetat, 55 bis 70 Gew.-% eines Acrylsäure-$C_{2-8}$-alkylesters und 3 bis 10 Gew.-% eines Acrylsäure-$C_{2-4}$-hydroxyalkylesters enthält und das nach Zumischen eines üblichen Vernetzers und dem Nitroglycerin zusätzlich durch Erwärmen und Entfernen von vorhandenen Lösungsmitteln vernetzt ist. Die spezielle erfindungsgemäße Klebemasse hat nicht nur eine hohe Aufnahmekapazität, sondern auch eine hohe und kontrollierbare Abgabefähigkeit für Nitroglycerin, so daß für die notwendige Freisetzungsmenge pro Tag die Freisetzungsfläche des Pflasters klein gehalten werden kann und so die Kosten des Pflasters sehr niedrig sind. Gleichzeitig wird durch die einfache Klebemasse das Herstellungsverfahren vereinfacht, der Zusatz weiterer Stoffe zur Erhöhung des transepidermalen Stofftransports eingespart und so die Kosten des Pflasters weiter gering gehalten und das Risoko von Hautirritationen vermieden.

EP 0 518 113 A1

Die vorliegende Erfindung betrifft ein Hautpflaster zur transdermalen Verabreichung von Nitroglycerin, bestehend aus einer Trägerfolie und einer Nitroglycerin enthaltenden Klebemasse auf Basis eines vernetzten Acrylat-Copolymerisates. Das Pflaster weist weiterhin eine Schutzfolie auf, die vor Gebrauch des Pflasters, d.h. vor Anbringen desselben auf die Haut durch Abziehen entfernt wird.

Hautpflaster zur transdermalen Verabreichung von Nitroglycerin sind zahlreich bekannt. Zum Beispiel beschreiben DE 2135533 und DE 3315272 Pflaster, die mehrschichtig aufgebaut sind und die Wirkstoffabgabe steuern. Nitroglycerin wird nach verschiedenen Mechanismen, sei es aus einem einschichtigen Reservoir durch eine Steuermembran (DE 2135533), sei es durch besondere Gestaltung des mehrschichtigen Reservoirs (DE 3315272), freigesetzt. Da die vielschichtigen Hautpflaster insbesondere in ihrer Herstellung recht teuer sind, hat man in jüngerer Vergangenheit Pflaster entwickelt, die neben der Trägerfolie aus einer einzigen Schicht aufgebaut sind. Um in genügendem Ausmaß Nitroglycerin aufnehmen und wieder in genügendem Maß Nitroglycerin an die Haut abegeben zu können, hat man hierbei verschiedene selbstklebende Haftklebemassen mit den verschiedensten Eigenschaften in bezug auf Wirkstoffaufnahmekapazität, Wirkstoffabgabe und Haftfähigkeit auf der Haut entwickelt. Als Beispiele hierfür seien genannt GB-A 2095108, DE-OS 3231400, GB-A 2086224, EP-A 0062682, EP 85903926.5, EP 86902978.5, EP 0285550, EP 0272562, US 4608249 und DE-PS 3200369. Je nach den eingesetzten Materialien und dem Vernetzungsgrad haben die Pflaster unterschiedliche Aufnahmekapazität und Abgabefähigkeit für Nitroglycerin und sind durch eine unterschiedliche Haftfähigkeit zur Haut gekennzeichnet. Unterschiedliche Hautverträglichkeit spielt ebenso eine erhebliche Rolle. Manche der Pflaster enthalten zusätzlich Stoffe zur Erhöhung des transepidermalen Stofftransports (sog. Resorptionsbeschleuniger).

Aufgabe der vorliegenden Erfindung ist die Schaffung eines Hautpflasters zur transdermalen Verabreichung von Nitroglycerin, das gekennzeichnet ist durch Einsatz eines Haftklebers, der nicht nur eine möglichst hohe Aufnahmekapazität, sondern auch eine hohe Abgabefähigkeit für Nitroglycerin besitzt, so daß für die notwendige Freisetzungsmenge pro Tag die Freisetzungsfläche des Pflaster klein gehalten werden kann und hierdurch die Kosten des Pflasters möglichst niedrig sind. Gleichzeitig soll durch Einsetzung eines speziellen Klebers das Herstellungsverfahren vereinfacht, seine Kosten gering und der Zusatz von Resorptionsbeschleunigern eingespart werden. Diese Vereinfachung der pharmazeutischen Formulierung verringert zugleich das Risiko von Hautirritationen und/oder einer unkontrollierbaren Veränderung der Nitroglycerinkonzentration in der Haftklebemasse, was mit der Penetration von Resorptionsbeschleunigern aus der Klebemasse in die Haut einhergehen kann.

Das erfindungsgemäße Hautpflaster zur transdermalen Verabreichung von Nitroglycerin, bestehend aus einer Trägerfolie, einer Nitroglycerin enthaltenden Klebemasse auf Basis eines vernetzten Acrylat-Vinylacetat-Copolymerisats und einer üblichen abziehbaren Schutzfolie ist dadurch gekennzeichnet, daß die das Nitroglycerin enthaltende Klebemasse dadurch erhalten ist, daß in einer ersten Stufe ein Gemisch aus 21 bis 40 Gew.-% Vinylacetat, 55 bis 70 Gew.-% eines Acrylsäure-$C_{2-8}$-alkylesters und 3 bis 10 Gew.-% eines Acrylsäure-$C_{2-4}$-hydroxyacrylesters, bei 100 Gew.-% Monomeren im Gemisch, in einem organischen Lösungsmittel einer radikalischen Polymerisation unterworfen wird, sodann in einer zweiten Stufe ein übliches Vernetzungsmittel in einem organischen Lösungsmittel und das Nitroglycerin in der für die beabsichtigte Anwendung des Pflasters notwendigen Menge, gegebenenfalls in einem organischen Lösungsmittel zugemischt wird und schließlich in einer dritten Stufe das erhaltene Gemisch bzw. das bestimmte Acrylat-Vinylacetat-Copolymerisat in einer zusätzlichen Stufe unter Erwärmen und Entfernen des eingesetzten organischen Lösungsmittels bzw. Lösungsmittelgemischs vernetzt wird und das enthaltene Nitroglycerin durch die nachträgliche und zusätzliche Vernetzung des besonderen Acrylat-Vinylacetat-Copolymerisats in besonderer Weise in die Klebemasse "eingebaut" wird. Das Acrylat-Vinylacetat-Copolymerisat hat eine relative Viskosität von 3,0 bis 4,2.

Bevorzugt enthält das Monomerengemisch neben Vinylacetat 2-Ethylhexylacrylat und Hydroxyethylacrylat. Bevorzugt ist die nachfolgende Vernetzung des besonderen Acrylat-Vinylacetat-Copolymerisatsmit einem Titansäureester bestehend aus Polybutyltitanat und/oder Titanacetylacetonat, insbesondere in einer Menge von 0,3 bis 3 Gew.-% hiervon, die Gewichtsprozente bezogen auf das Gewicht des Copolymerisats, durchgeführt.

Das Verfahren zur Herstellung des erfindungsgemäßen Pflasters ist dadurch gekennzeichnet, daß eine Nitroglycerin in der für die beabsichtigte Anwendung des Pflasters notwendigen Menge und einen üblichen Vernetzer oder ein übliches Vernetzergemisch enthaltende Lösung eines durch radikalische Polymerisation eines Monomerengemisches bestehend aus 21 bis 40 Gew.-% Vinylacetat, 55 bis 70 Gew.-% eines Acrylsäure-$C_{2-8}$-alkylesters und 3 bis 10 Gew.-% eines Acrylsäure-$C_{2-4}$-hydroxyalkylesters erhaltenen Copolymerisats auf die Schutzfolie des Pflasters in der gewünschten Schichtdicke aufgetragen wird und das Lösungsmittel oder Lösungsmittelgemisch unter Erwärmen entfernt und so die zusätzliche Vernetzung des besonderen Acrylat-Vinylacetat-Copolymerisats durchgeführt wird.

Vorzugsweise ist das Verfahren dadurch gekennzeichnet, daß das Acrylat-Vinylacetat-Copolymerisat, Nitroglycerin und Vernetzer gelöst sind in einem Lösungsmittel, das 20 bis 40 Gew.-% Ethanol oder eines Ethanol-Methanol-Gemisches enthält, mit einem Feststoffanteil, bestehend aus 40 bis 60 Gew.-% des Gemischs aus dem besonderen Acrylat-Vinylacetat-Copolymerisat, Vernetzer und dem Nitroglycerin.

Ausführungsbeispiel

Herstellungsverfahren für Hautpflaster zur transdermalen Anwendung von Nitroglycerin gemäß vorliegender Erfindung, die Mengenangaben bezogen auf eine Ansatzgröße von 100 m².

Zu 16,00 kg einer 40 %-igen Lösung (G/G) des Acrylat-Vinylacetat-Copolymerisates werden unter intensiver Durchmischung 4,00 kg Nitroglycerin in öliger Form langsam zugeführt. Anschließend wird die Mischung durch Rühren homogenisiert. Es resultiert eine 20 %-ige (G/G) Lösung von Nitroglycerin in dieser Kleberlösung.

Das Acrylat-Vinylacetat-Copolymerisat wird wie folgt hergestellt:

Von der Gesamtmenge von 112 g Vinylacetat, 270 g 2-Ethylhexylacrylat, 20 g Hydroxyethylacrylat, 1,4 g Azodiisobutyronitril und 407 g Ethylacetat werden 112 g Vinylacetat, 39 g 2-Ethylhexylacrylat, 3 g Hydroxyethylacrylat und 0,5 g Azodiisobutyronitril zu 115 g Ethylacetat zugegeben und bis zum Rückfluß erhitzt. Der Restanteil der Bestandteile wird über eine Zeitdauer von 4 Stunden unter konstantem Rückfluß zugegeben. Nach Beendigung der Polymerisation wird die Mischung auf Raumtemperatur abgekühlt. Die resultierende Kleberpolymerlösung hat eine Viskosität von 5300 mPa.s bei 25ºC, gemessen mit einem Brookfield-Viskometer, einen Feststoffanteil von 47,9 % und die relative Viskosität beträgt 3,1.

Zu dieser Lösung werden 1,35 Titanacetylacetonat und genügend Ethanol oder Ethanol-Ethylacetat-Mischung zugegeben, um den Feststoffgehalt im Produkt auf 40 % einzustellen.

Beipiel 1

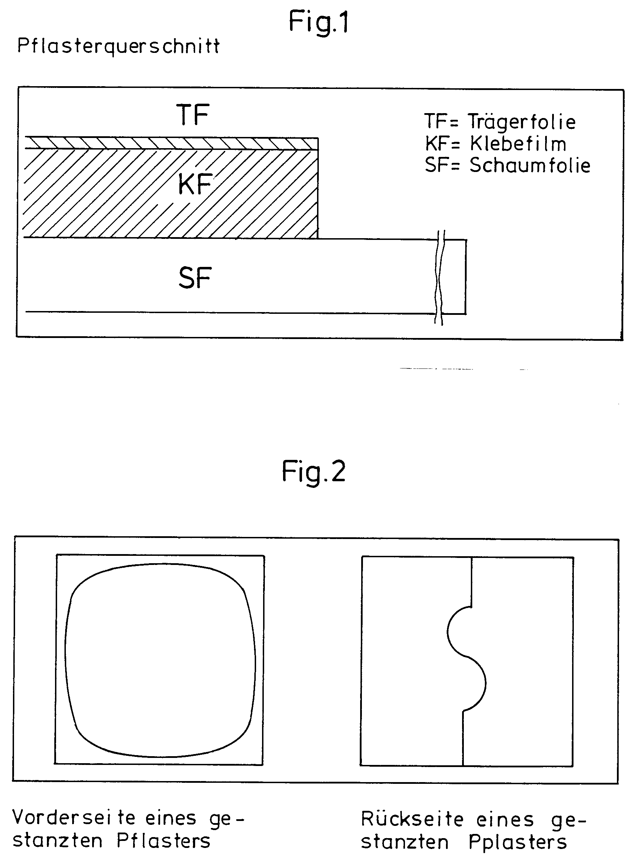

Die oben genannte 20 % (G/G) Nitroglycerin enthaltende Kleberlösung wird auf eine 100 μm dicke silikonisierte Polyesterfolie aufgetragen, so daß nach dem Entfernen des Lösungsmittels ein Film mit einem Flächengewicht von 92 g/m² resultiert. Dieser Film wird mit einer 19 μm dicken Polyesterfolie abgedeckt und zu Pflastern mit einer Kontaktfläche von 16 qcm gestanzt (Abb. 1 und 2). Ein so hergestelltes Hautpflaster mit einem Gewicht von 420 mg enthält 55 mg Nitroglycerin.

Zur Beurteilung des Wirkstoffliberationsverhaltens in vitro wird ein Pflaster mit einer ausgestanzten Fläche von 3,14 qcm in einer modifizierten Franz-Diffusionszelle (vgl. Chien, Yie W., Drugs of Today Vol. 23, No. 11 (1987) 625 - 646) auf einer Hautpräparation haarloser Mäuse befestigt.

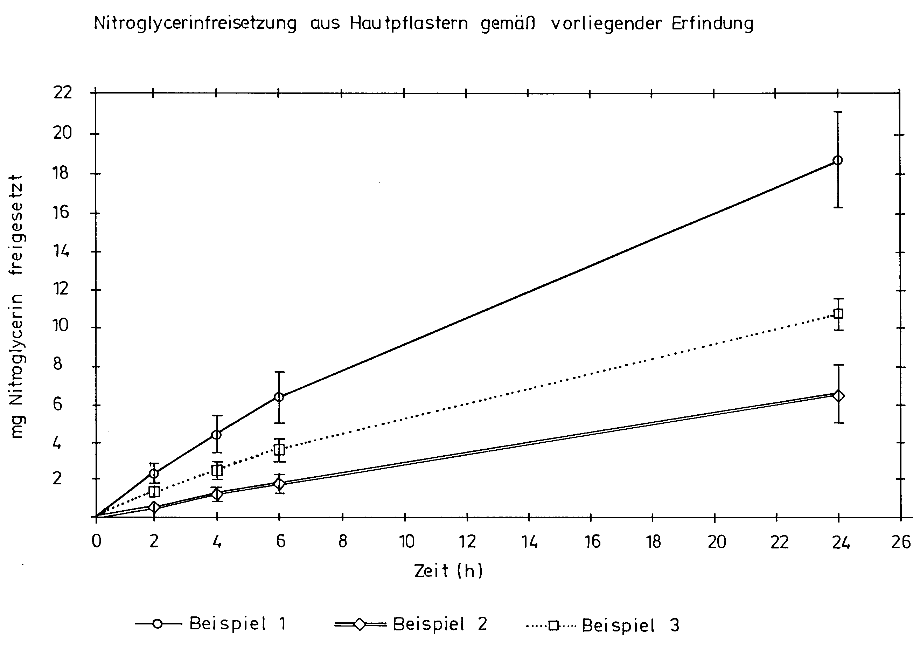

Unmittelbar anschließend wird die Zelle mit 18,00 ml isotonischer Phosphatpufferlösung (32 ± 0,5ºC) luftblasenfrei befüllt und auf 32 ± 0,5ºC thermostatisiert. Nach 2, 4, 6 und 24 Stunden wird das Freisetzungsmedium durch frische auf 32 ± 0,5ºC thermostatisierte Lösung ersetzt. Die entnommene Lösung wird HPLC-chromatographisch ( = hochleistungsflüssigkeitschromatographisch (Lit.: Pharm.Biol. 4, 32 (1981)) auf ihren Nitroglyceringehalt untersucht. Das nach dieser Methode gemessene Freisetzungsprofil für ein 16 qcm großes Pflaster ist in Abb. 3 wiedergegeben.

Die mittleren Nitroglycerin-Freisetzungsraten (± S.D.) betrugen (n = 3):

| nach 2 Stunden | 2,32 ± 0,56 mg/16 qcm |
|---|---|
| nach 4 Stunden | 4,42 ± 1,00 mg/16 qcm |
| nach 6 Stunden | 6,43 ± 1,33 mg/16 qcm |
| nach 24 Stunden | 18,74 ± 2,43 mg/16 qcm |

Beispiel 2

Der oben genannten 20 % (G/G) Nitroglycerin enthaltenden Kleberlösung werden zusätzlich 0,8 % (G/G) Titanacetylacetonat (Hersteller: Dynamit Nobel Nederland B.V., 75 %-ige (G/G) Lösung in Isopropanol), bezogen auf einen 40 %-igen (G/G) Feststoffanteil der Polyacrylatkleberlösung zuzüglich Nitroglycerin, zugesetzt und das Gemisch homogenisiert. Diese Lösung wird auf eine 100 μm dicke silikonisierte Polyesterfolie aufgetragen, so daß nach dem Entfernen des Lösungsmittels ein Film mit einem Flächengewicht von 93 g/m² resultiert. Dieser Film wird mit einer 19 μm dicken Polyesterfolie abgedeckt und zu Pflastern mit einer Kontaktfläche von 16 qcm gestanzt (Abb. 1 und 2). Ein so hergestelltes Hautpflaster mit einem Gewicht von 420 mg enthält 55 mg Nitroglycerin.

Die Wirkstofffreisetzung in vitro wurde entsprechend der Methode in Beispiel 1 durchgeführt. Das entsprechende Freisetzungsprofil ist ebenfalls in Abb. 3 graphisch wiedergegeben.

Die mittleren Nitroglycerin-Freisetzungsraten (± S.D.) betrugen (n = 3):

| | |
|---|---|
| nach 2 Stunden | 0,54 ± 0,20 mg/16 qcm |
| nach 4 Stunden | 1,20 ± 0,37 mg/16 qcm |
| nach 6 Stunden | 1,78 ± 0,53 mg/16 qcm |
| nach 24 Stunden | 6,60 ± 1,56 mg/16 qcm |

Beispiel 3

Die oben genannte 20 % (G/G) Nitroglycerin enthaltende Kleberlösung wird auf eine 100 $\mu$m dicke silikonisierte Polyesterfolie aufgetragen, so daß nach dem Entfernen des Lösungsmittels ein Film mit einem Flächengewicht von 64 g/m$^2$ resultiert. Dieser Film wird mit einer 19 $\mu$m dicken Polyesterfolie abgedeckt und zu Pflastern mit einer Kontaktfläche von 16 qcm gestanzt (Abb. 1 und 2). Ein so hergestelltes Hautpflaster mit einem Gewicht von 360 mg enthält 40 mg Nitroglycerin. Die wirkstofffreisetzung in vitro wurde entsprechend der Methode in Beispiel 1 durchgeführt. Das entsprechende Freisetzungsprofil ist ebenfalls in Abb. 3 graphisch wiedergegeben.

Die mittleren Nitroglycerin-Freisetzungsraten (± S.D.) betrugen (n = 3):

| | |
|---|---|
| nach 2 Stunden | 1,27 ± 0,29 mg/16 qcm |
| nach 4 Stunden | 2,48 ± 0,48 mg/16 qcm |
| nach 6 Stunden | 3,56 ± 0,60 mg/16 qcm |
| nach 24 Stunden | 10,79 ± 0,82 mg/16 qcm. |

**Patentansprüche**

1. Hautpflaster zur transdermalen Verabreichung von Nitroglycerin, bestehend aus einer Trägerfolie, einer Nitroglycerin enthaltenden Klebemasse auf Basis eines Acrylat-Vinylacetat-Copolymerisats und einer vor Gebrauch entfernbaren üblichen Schutzfolie, **dadurch gekennzeichnet,** daß die das Nitroglycerin enthaltende Klebemasse hergestellt ist durch:
   1) radikalische Polymerisation eines Gemisches von 21 bis 40 Gew.-% Vinylacetat, 55 bis 70 Gew.-% eines Acrylsäure-$C_{2-8}$-alkylesters und 3 bis 10 Gew.-% eines Acrylsäure-$C_{2-4}$-hydroxyalkylesters, bezogen auf 100 Gew.-% des eingesetzten Monomerengemisches, in einem organischen Lösungsmittel,
   2) Zumischen eines üblichen Vernetzers in einem organischen Lösungsmittel und des Nitroglycerins in der für die Anwendung des Pflasters notwendigen Menge und
   3) Vernetzung des erhaltenen Gemischs unter Erwärmung und Entfernung des eingesetzten Lösungsmittels oder Lösungsmittelgemischs.

2. Hautpflaster gemäß Anspruch 1, dadurch gekennzeichnet, daß der Acrylsäure-$C_{2-4}$-hydroxyalkylester Hydroxyethylacrylat ist.

3. Hautpflaster gemäß einem der Ansprüche 1 und 2, dadurch gekennzeichnet, daß der Acrylsäure-$C_{2-8}$-alkylester neben 2-Hydroxyethylacrylat Ethylacrylat ist.

4. Hautpflaster gemäß einem der Ansprüche 1 bis 3, dadurch gekennzeichnet, daß der Vernetzer ein Titansäureester oder ein Titansäureestergemisch ist.

5. Hautpflaster gemäß Anspruch 4, dadurch gekennzeichnet, daß zur Vernetzung 0,3 bis 3 Gew.-% eines Titansäureesters oder Titansäureestergemischs eingesetzt werden, wobei die Gewichtsprozent bezogen sind auf das Gewicht des Vernetzer enthaltenden, durch radikalische Polymerisation erhaltenen Copolymerisats.

6. Hautpflaster gemäß einem der Ansprüche 4 und 5, dadurch gekennzeichnet, daß der Vernetzer Titanacetylacetonat und/oder Polybutyltitanat ist.

7. Verfahren zur Herstellung eines Pflasters gemäß einem der Ansprüche 1 bis 6, dadurch gekennzeichnet, daß ein das Nitroglycerin in der zur Anwendung des Pflasters notwendigen Menge und einen Vernetzer enthaltende Lösung eines Acrylat-Vinylacetat- Copolymerisats, hergestellt durch radikalische Polymerisation eines Monomerengemisches bestehend aus 21 bis 40 Gew.-% Vinylacetat, 55 bis 70 Gew.-% eines Acrylsäure-$C_{2-8}$-alkylesters und 3 bis 10 Gew.-% eines Acrylsäure-$C_{2-4}$-hydroxyalkylesters, bezogen auf 100 Gew.-% des eingesetzten Monomerengemisches, in einem organischen Lösungsmittel, auf die Schutzfolie des Pflasters in der gewünschten Schichtdicke aufgetragen wird, das Lösungsmittel oder Lösungsmittelgemisch unter Erwärmen entfernt wird, und so die Vernetzung des besonderen Acrylat-Vinylacetat-Copolymerisats durchgeführt wird und sodann die Trägerfolie aufgebracht wird und das Pflaster auf die gewünschte Größe zugeschnitten und/oder gestanzt wird.

8. Verfahren gemäß Anspruch 7, dadurch gekennzeichnet, daß das besondere Acrylat-Vinylacetat-Copolymerisat, das Nitroglycerin und der Vernetzer gemeinsam in einem Lösungsmittelgemisch gelöst sind, das 20 bis 40 Gew.-% Ethanol oder eines Ethanol-Methanol-Gemisches enthält, und sein Feststoffanteil, bestehend aus dem besonderen Acrylat-Vinylacetat-Copolymerisat, Vernetzer und Nitroglycerin, 40 bis 60 Gew.-% beträgt.

## Fig.1

Pflasterquerschnitt

TF

KF

SF

TF= Trägerfolie
KF= Klebefilm
SF= Schaumfolie

## Fig.2

Vorderseite eines gestanzten Pflasters

Rückseite eines gestanzten Pplasters

Nitroglycerinfreisetzung aus Hautpflastern gemäß vorliegender Erfindung

mg Nitroglycerin freigesetzt

Zeit (h)

—o— Beispiel 1

⟨◇⟩ Beispiel 2

······□······ Beispiel 3

Europäisches
Patentamt

**EUROPÄISCHER RECHERCHENBERICHT**

Nummer der Anmeldung

EP 92 10 8798

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DERWENT FILE SUPPLIER & BASE WPIL, AN=88-033094 [05], Derwent Publications Ltd, London, GB; & JP-A-62 292 877 (NIPPON SHOKUBAI KAGAKU) 19-12-1987 * Zusammenfassung * --- | 1-8 | A 61 K 31/21 A 61 K 9/70 |
| D,Y | EP-A-0 285 550 (SEKISUI KAGAKU) * Seite 4, Absatz 1; Ansprüche * --- | 1-8 | |
| D,Y X | EP-A-0 272 562 (LTS LOHMANN THERAPIE-SYST.) * Seite 3, Zeilen 40-55; Seite 4, Zeilen 1-6,18-27; Seite 5, Zeile 20 * --- | 1-8 | |
| Y | GB-A-2 086 224 (NITTO ELECTRIC INDUSTRIAL) * Seite 2, Zeilen 23-24,31-35,38-42; Ansprüche * --- | 1-8 | |
| P,X | EP-A-0 435 199 (NITTO DENKO CORP.) * Seite 3, Zeilen 19-55; Seite 5, Zeilen 13-23; Beispiel 7; Ansprüche 1,5-8 * --- | 1-8 | **RECHERCHIERTE SACHGEBIETE (Int. Cl.5)** A 61 K |
| A | CHEMICAL ABSTRACTS, Band 97, Nr. 12, September 1982, Seite 403, Zusammenfassung Nr. 98386f, Columbus, Ohio, US; & JP-A-82 107 155 (NITTO ELECTRIC INDUSTRIAL CO., LTD) 03-07-1982 * Zusammenfassung * --- | 1-8 | |
| A | EP-A-0 427 877 (NITTO DENKO CORP.) * Beispiel 1; Ansprüche * --- -/- | 1-8 | |

Der vorliegende Recherchenbericht wurde für alle Patentansprüche erstellt

| Recherchenort | Abschlußdatum der Recherche | Prüfer |
|---|---|---|
| DEN HAAG | 24-07-1992 | BERTE M.J. |

Europäisches
Patentamt

# EUROPÄISCHER RECHERCHENBERICHT

Nummer der Anmeldung

EP 92 10 8798

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,A | EP-A-0 450 986 (SEKISUI KAGAKU KOGYO) * Seite 3, Zeile 52 - Seite 4, Zeile 4,25-27; Ansprüche * --- | 1-8 | |
| D,A | WO-A-8 606 281 (RIKER LABS.) * Seite 5, Zeile 18 - Seite 6, Zeile 13; Ansprüche * --- | 1-8 | |
| D,A | EP-A-0 062 682 (NICHIBAN CO.) * Ansprüche * ----- | 1-8 | |

RECHERCHIERTE
SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| DEN HAAG | 24-07-1992 | BERTE M.J. |